# EUROPÄISCHE PATENTSCHRIFT

(11) **EP 0 475 419 B1**
(45) Veröffentlichungstag und Bekanntmachung des Hinweises auf die Patenterteilung: **05.02.1997**
(21) Anmeldenummer: 91115499.5
(22) Anmeldetag: 12.09.1991
(51) Int. Cl.: A61F 13/15

(54) **Wegwerbekleidung und Verfahren zum Anbringen von elastischen Elementen um deren Beinöffnungen**
Disposable clothing, and procedure for applying elastic elements around its leg openings
Vêtements jetable, et procédé pour mettre des éléments élastiques autour des ouvertures de jambes

(30) Priorität: 13.09.1990 JP 243417/90; 17.04.1991 JP 113986/90
(43) Veröffentlichungstag der Anmeldung: 18.03.1992
(73) Patentinhaber: UNI-CHARM CORPORATION, Kawanoe-shi Ehime-ken (JP)
(72) Erfinder: Igaue, Takamitsu, Kawanoe-shi, Ehime-ken (JP); Nomura, Hironori, Iyomishima-shi, Ehime-ken (JP); Ohnishi, Hirofumi, Iyomishima-shi, Ehime-ken (JP); Matsura, Yoshinori, Kanonji-shi, Kagawa-ken (JP); Sasaki, Tohru, Kawanoe-shi, Ehime-ken (JP); Shimakawa, Taiji, Kanonji-shi, Kagawa-ken (JP); Yamamoto, Hiroki, Mitoyo-gun, Kagawa-kenn (JP)
(74) Vertreter: Sperling, Rüdiger, Dipl.-Ing.

(56) Entgegenhaltungen:
- EP-A- 0 048 010
- EP-A- 0 405 575
- EP-A- 0 421 473
- US-A- 4 430 086

## Beschreibung

### DER ERFINDUNG ZUGRUNDELIEGENDER STAND DER TECHNIK

Die Erfindung betrifft eine Wegwerfbekleidung, die um kreisbogenförmige Beinöffnungen, die jeweils an gegenüberliegenden Seiten eines zwischen Vorder- und Hinterteilen einer oberen und einer unteren Lage begrenzten Schrittbereichs gebildet sind, mit elastischen Elementen versehen ist, die jeweils mehrere Einzelelemente umfassen, wobei diese in gewisser Anzahl vorhandenen elastischen Einzelelemente für jede der Beinöffnungen so angeordnet ist, daß die elastischen Einzelelemente in ihrem in Längsrichtung mittleren Bereich den größten Abstand voneinander haben und die Abstände vom mittleren Bereich zu den in Längsrichtung gegenüberliegenden Enden hin nach und nach verringert sind.

Wird das Kleidungsstück an die Haut des Trägers angelegt, so kommt es oft zum Austreten von Exkrementen entlang etwa der unteren Hälften der jeweiligen Beinöffnungen. Es ist daher bevorzugt, die elastischen Elemente in besonderem Ausmaß entlang etwa dieser unteren Hälfte jeder Beinöffnung anzuordnen, so daß insbesondere in diesem Abschnitt das Kleidungsstück dicht an der Haut des Trägers anliegen kann.

Bei den bisher vorgeschlagenen herkömmlichen Kleidungsstücken dieser Art, die praktisch verwendet wurden, wie auch bei den Verfahren oder Vorrichtungen zu deren Herstellung wurde die extensive Anordnung der elastischen Elemente im besonderen Ausmaß entlang etwa der unteren Hälfte der Beinöffnungen nicht berücksichtigt und es wurde weder ein Verfahren noch eine Vorrichtung zum Erzielen einer solchen Anordnung vorgeschlagen.

Aus der EP 0 421 473 A2 ist eine Wegwerfwindel gemäß dem Oberbegriff des Anspruchs 1 bekannt, welche an den Beinöffnungen elastische Elemente aufweist, die im mittleren Bereich einen größeren Abstand voneinander aufweisen als an den jeweiligen gegenüberliegenden Enden. Nachteilig an diesen elastischen Elementen an den Beinöffnungen ist, daß produktionsbedingt die Dehnspannung konstant oder sogar im Bereich der Enden der elastischen Elemente erhöht ist. Dies führt an den Enden der elastischen Elemente zu unangenehmen Druckstellen bzw. zum Einschneiden in die Haut.

Der Erfindung liegt die Aufgabe zugrunde, Wegwerfbekleidung der genannten Art so zu verbessern, daß entlang der unteren Hälfte jeder Beinöffnung das Kleidungsstück dicht an der Haut des Trägers anliegen kann. Des weiteren werden zwei Verfahren zum Anbringen von elastischen Elementen an Wegwerfbekleidung angegeben. Der Oberbegriff des Anspruchs 2 ist aus der EP-A-0 048 010 bekannt. Der Oberbegriff des Anspruchs 3 ist aus der EP-A-0 405 575 bekannt.

### BESCHREIBUNG DER ERFINDUNG

### Produkt:

Wegwerfbekleidungsstücke sind um kreisbogenförmige Beinöffnungen, die an jeweils gegenüberliegenden Seiten eines Schrittbereichs angeordnet sind, der zwischen Vorder- und Hinterteil oberer und unterer Lagen gebildet ist, mit elastischen Elementen versehen, die jeweils mehrere elastische Einzelelemente umfassen. Diese Anzahl von elastischen Einzelelementen für jede der Beinöffnungen ist so angeordnet, daß die Einzelelemente in möglichst großem Abstand zueinander angeordnet sind, der sich von ihren Mittelbereichen zu ihren in Längsrichtung gegenüberliegenden Enden in Längsrichtung verringert.

Die Dehnspannung ihrer in Längsrichtung mittleren Bereiche ist dabei auf die in Längsrichtung gegenüberliegenden Enden zu nach und nach verringert.

### Verfahren:

Fadenähnliche endlose elastische Elemente, die jeweils mehrere Einzelelemente umfassen, werden in gespanntem Zustand von Gruppen von parallel im Abstand zueinander angeordneten Führungen gehalten, die an jeweiligen Traversiereinrichtungen nahe an deren vorderen Enden vorgesehen sind. Die Traversiereinrichtungen werden quer zu einer kontinuierlich zugeführten Endlosbahn, die als Werkstoff für die obere oder untere Lage jedes Bekleidungsstückes dient, hin und her geführt, so daß die elastischen Elemente mit der Endlosbahn verbunden werden können, wobei die elastischen Einzelelemente parallel zueinander in Kurven gelegt werden.

Zum Verbinden der endlosen elastischen Elemente mit der Endlosbahn kann auf diese im Voraus ein Klebstoff aufgebracht werden oder auf die endlosen elastischen Elemente kann Klebstoff aufgetragen werden, während diese von der Traversiereinrichtung geführt und auf der Endlosbahn angeordnet werden.

Die übrigen Einzelteile des Kleidungsstückes werden schichtweise auf die Endlosbahn aufgebracht, die mit den fadenähnlichen endlosen elastischen Elementen verbunden wurde. Anschließend wird die Endlosbahn gemeinsam mit den fadenähnlichen endlosen elastischen Elementen in Bereichen quer geschnitten, in denen die fadenähnlichen endlosen elastischen Elemente nicht mit der Endlosbahn verbunden wurden, wodurch sich die nicht verbundenen Abschnitte der fadenähnlichen endlosen elastischen Elemente aufgrund ihrer eigenen Dehnspannung zusammenziehen.

Gemäß dem vorliegenden Verfahren halten die jeweiligen Gruppen von parallel im Abstand zueinander angeordneten Führungen der Traversiereinrichtung die fadenähnlichen endlosen elastischen Elemente, die jeweils mehrere Einzelelemente umfassen, in gespanntem Zustand und werden quer zur Endlosbahn hin und her bewegt, um so die fadenähnlichen endlosen elastischen Elemente auf der Endlosbahn in Kurven mit vorgegebener Wellenfrequenz zu führen. Entsprechend werden die fadenähnlichen endlosen elastischen Elemente mit der Endlosbahn in der Weise verbunden, daß die jeweils in gewisser Anzahl vorhandenen Einzelelemente parallel zueinander in Kurven gelegt werden, wobei sie in ihren in Längsrichtung mittleren Bereichen einen größeren Abstand zueinander haben als an den in Längsrichtung gegenüberliegenden Enden (der Klebebereiche der einzelnen Kleidungsstücke). Diese in Kurven verlaufenden Abschnitte der fadenähnlichen endlosen elastischen Elemente, die mit der Endlosbahn verbunden wurden, wirken als die fadenähnlichen elastischen Elemente um die jeweiligen Beinöffnungen der fertiggestellten Kleidungsstücke.

### KURZE BESCHREIBUNG DER ZEICHNUNGEN

- Fig. 1: zeigt eine perspektivische Darstellung eines mittels einer Ausführungsform des Verfahrens gemäß der vorliegenden Erfindung herzustellenden Kleidungsstückes;
- Fig. 2: zeigt eine aueinandergezogene perspektivische Darstellung dieses Kleidungsstückes;
- Fig. 3: zeigt eine schematische perspektivische Darstellung einer Traversiereinrichtung, die zur Durchführung eines Vorganges zum Aufbringen der fadenähnlichen endlosen elastischen Elemente auf eine Endlosbahn dient;
- Fig. 4: zeigt eine schematische Draufsicht, die verdeutlicht, wie die fadenähnlichen endlosen elastischen Elemente unter Verwendung der Traversiereinrichtung auf die Endlosbahn aufgebracht werden;
- Fig. 5: zeigt eine perspektivische Darstellung einer teilweisen Veränderung der in Fig. 3 gezeigten Traversiereinrichtung; und
- Fig. 6: zeigt eine Draufsicht zur Darstellung einer teilweisen Veränderung der in Fig. 4 gezeigten Anordnung der fadenähnlichen endlosen elastischen Elemente.

### BEVORZUGTE AUSFÜHRUNGSFORM DER ERFINDUNG

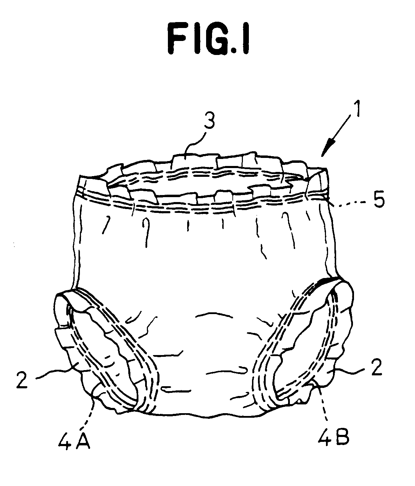

Fig. 1 zeigt eine perspektivische Darstellung eines Beispiels für ein durch ein Verfahren der vorliegenden Erfindung hergestelltes Kleidungsstück. Das Kleidungsstück 1 verfügt über zwei Beinöffnungen 2 und eine Hüftöffnung 3, die jeweils mit elastischen Elementen 4A, 4B bzw. 5 versehen sind.

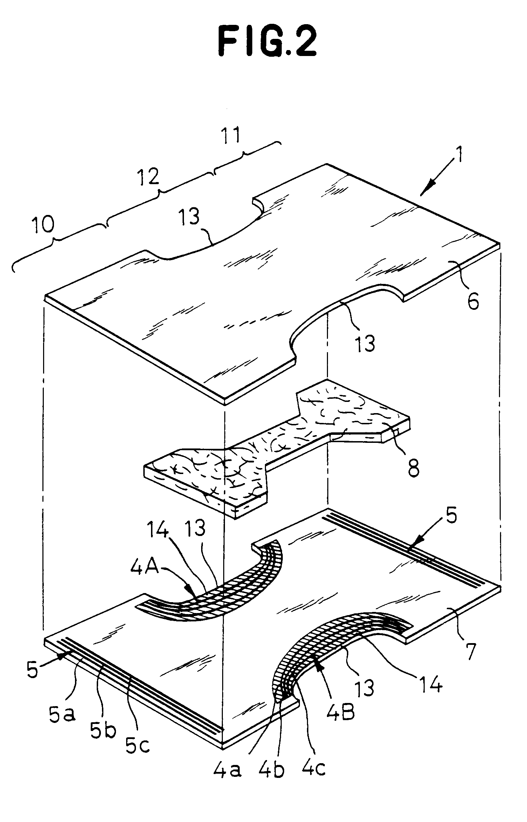

Fig. 2 ist eine auseinandergezogene perspektivische Darstellung des Kleidungsstücks 1. Das Kleidungsstück 1 umfaßt eine aus einem flüssigkeitsdurchlässigen Vliesstoff, der sowohl in der Länge als auch in der Breite dehnbar ist, gefertigte obere Lage 6, eine aus einem flüssigkeitsundurchlässigen Vliesstoff, der sowohl in der Länge als auch in der Breite dehnbar ist, gefertigte untere Lage 7, einen matten- oder plattenartigen Flüssigkeitsabsorptionskern 8, der im wesentlichen aus lockerer Fasermasse besteht, sowie die fadenähnlichen elastischen Elemente 4A, 4B und 5, die jeweils um die Beinöffnungen bzw. die Hüftöffnung angeordnet sind. Ein Schrittbereich 12 erstreckt sich zwischen Vorder- und Hinterteil 10, 11 der oberen und unteren Lage und ist entlang den einander gegenüberliegenden Seitenrändern mit konkav gekrümmten Ausnehmungen 13 versehen, die den Beinöffnungen 2 entsprechen. Wenn auch nicht dargestellt, so ist es doch möglich, eine untere Lage 7 zu verwenden, die einen in Länge und Breite dehnbaren flüssigkeitsdurchlässigen Vliesstoff und eine in Länge und Breite dehnbare, flüssigkeitsundurchlässige Kunststoff- oder Gummifolie umfaßt, die mit Unterbrechungen mittels Klebstoff mit der Innenseite des flüssigkeitsdurchlässigen Vliesstoffes verbunden ist. Die vorstehende Anordnung erlaubt nicht nur, die untere Lage 7 so zu gestalten, daß flüssige Ausscheidungen zuverlässig am Durchdringen derselben gehindert werden, sondern verbessert auch, wenn wenigstens der äußere Umfangsbereich der Folie mit Unterbrechungen mittels Klebstoff mit der oberen Lage 6 verbunden ist, die Dehnbarkeit der oberen und unteren Lagen 6 und 7 als die Hauptmaterialien des Kleidungsstücks, und verbessert so in der Folge die Dehnspannungseigenschaften, womit eine weitere Erhöhung der Paßgenauigkeit des Kleidungsstücks 1 am Körper des Trägers erzielt wird.

Jedes der um die jeweiligen Beinöffnungen vorgesehenen elastischen Elemente 4A und 4B umfaßt mehrere Fäden 4a, 4b, 4c aus Naturgummi oder synthetischem Gummi, die parallel zueinander mit Abständen verlaufen, die von den Mittelbereichen in Längsrichtung (entsprechend dem Schrittbereich) nach und nach auf die jeweils in Längsrichtung gegenüberliegenden Enden zu abnehmen, wobei ihre Dehnspannung von ihren in Längsrichtung mittleren Bereichen auf die in Längsrichtung gegenüberliegenden Enden zu nach und nach verringert ist. Die elastischen Elemente 4A und 4B sind mit der unteren Lage 7 jeweils entlang einer zugehörigen, in einer Kurve verlaufenden Klebezone 14 verbunden, die entlang der zugehörigen, konkav gekrümmten Ausnehmung 13 verläuft und mit Klebstoff versehen wurde. In ähnlicher Weise umfaßt das elastische Element 5 mehrere Fäden 5a und 5b aus Naturgummi oder synthetischem Gummi, die mit der unteren Lage 7 entlang einer nicht dargestellten Klebezone, die durch auf die untere Lage aufgetragenen Klebstoff gebildet ist, oder mittels auf die Gummifäden selbst aufgetragenem Klebstoff verbunden sind.

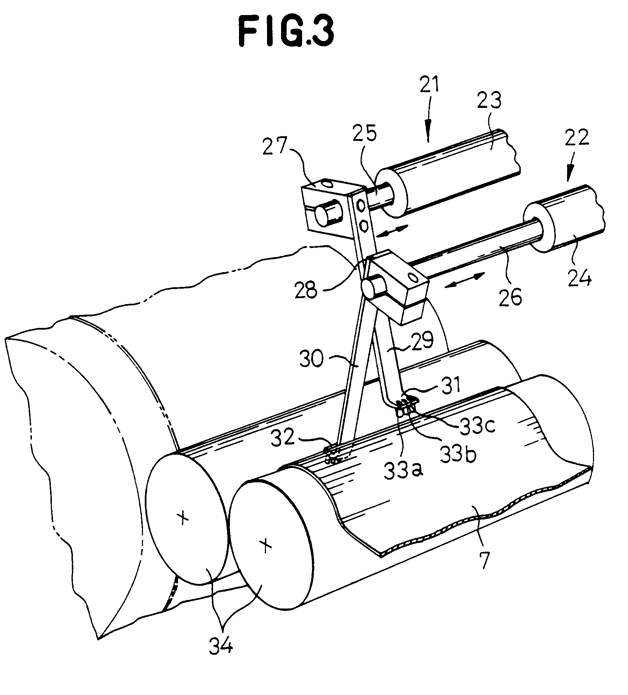

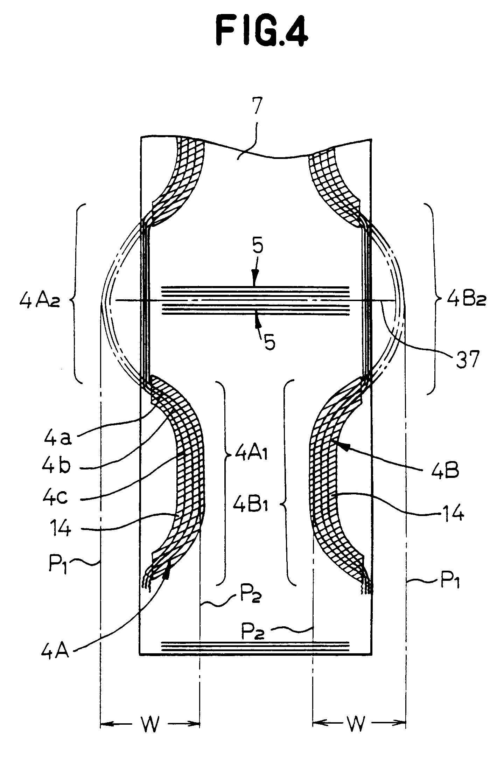

Fig. 3 ist eine schematische perspektivische Darstellung zur Erläuterung der Traversiereinrichtung, die zum Aufbringen der fadenähnlichen endlosen elastischen Elemente auf die als Material für die einzelnen unteren Lagen dienende Endlosbahn 7 in der vorstehend beschriebenen Weise dient und Fig. 4 ist eine Draufsicht zur Erläuterung der Weise, wie die fadenähnlichen endlosen elastischen Elemente unter Verwendung der Traversiereinrichtung auf die Endlosbahn aufzubringen sind. Die Vorrichtung zur Durchführung dieses Verfahrens ist mit Ausnahme der dargestellten Traversiereinrichtung bekannt. Dieses Verfahren kann auch unter Verwendung einer Vorrichtung zur Herstellung von Wegwerfwindeln nach dem Stand der Technik durchgeführt werden, beispielsweise der von der Anmelderin in der EP 0 405 575 A1 aufgezeigten Vorrichtung.

Wie Fig. 3 zeigt, umfassen die Traversiereinrichtungen 21, 22 Tragzylinder 23, 24, die nahe an und parallel zu zwei Klemmwalzen 34, 34 angeordnet sind, jeweils in die Tragzylinder 23, 24 eingesetzte Schubstangen 25, 26, jeweils an den vorderen Enden der Schubstangen 25, 26 befestigte Tragblöcke 27, 28 sowie Führungshebel 29, 30, die jeweils von den Tragblöcken 27, 28 herabhängen. Jeder der Führungshebel 29, 30 ist an seinem umgebogenen unteren Ende 31, 32 mit mehreren kleinen zylindrischen Führungen 33a, 33b, 33c versehen. Diese Führungen 33a, 33b, 33c sind in Richtung der Bewegung des zugehörigen Führungshebels im Abstand voneinander angeordnet. Die umgebogenen Enden 31, 32 befinden sich nahe an den Umfangsflächen der jeweiligen Klemmwalze 34, 34. Die Schubstangen 25, 26 werden von nicht gezeigten Kurvenwalzen-Traversiermechanismen gesteuert, die mit den hinteren Enden der jeweiligen Schubstange 25, 26 in einer später beschriebenen Weise verbunden sind.

Wie in Fig. 4 gezeigt, ist auf die Endlosbahn 7 mit Unterbrechungen in vorgegebenen Abständen entlang gegenüberliegenden Seitenrändern Klebstoff aufgetragen, um so aufeinanderfolgende, bezüglich der zugehörigen Seitenränder in einer konkaven Kurve verlaufende Klebezonen 14 zu bilden, während die Endlosbahn 7 mit vorgegebener Geschwindigkeit in Längsrichtung bewegt wird. Währenddessen werden die Gummifäden 4a, 4b, 4c, die die jeweiligen fadenähnlichen elastischen Elemente 4A, 4B bilden, durch die jeweiligen Führungen 33a, 33b, 33c der Traversiereinrichtung 21, 22 geführt, während die Gummifäden 4a, 4b, 4c mit einem vorgegebenen Dehnungsgrad gedehnt werden. In diesem Zustand werden die Führungshebel 29, 30 quer über den jeweiligen Seitenrändern der Endlosbahn 7 hin und her bewegt, so daß die Gummifäden 4a, 4b, 4c der jeweiligen Gruppe (d. h. der fadenähnlichen elastischen Elemente 4A, 4B) sinuskurvenartige Kurven beschreiben, die den jeweiligen, einander symmetrischen Klebezonen 14 folgen.

Die fadenähnlichen endlosen elastischen Elemente 4A, 4B werden jeweils aus einer ersten Position P₁ zu einer zweiten Position P₂ und von der zweiten Position P₂ zur ersten Position P₁ über eine Breite W bewegt, wobei während der Bewegung von der ersten Position P₁ zur zweiten Position P₂ der Grad ihrer Dehnung geringfügig erhöht wird und sich ihre Dehnspannungen entsprechend erhöhen. Genauer ausgedrückt werden die fadenähnlichen endlosen elastischen Elemente 4A, 4B, die mit einem bestimmten Dehnungsgrad gestreckt linear entlang der Längsrichtung der Endlosbahn 7 laufen, von den Führungshebeln 29, 30 zwangsweise quer zur Endlosbahn 7 geleitet und der Widerstand der jeweiligen elastischen Elemente 4A, 4B gegen diese zwangsweise Richtungsänderung verursacht die Dehnung der elastischen Elemente 4A, 4B bei ihrer Bewegung von der ersten Position P₁ zur zweiten Position P₂. In diesem Zusammenhang versteht es sich, daß der Grad der Dehnung von der Geschwindigkeit abhängig ist, mit der die fadenähnlichen endlosen elastischen Elemente 4A, 4B in Längsrichtung der Endlosbahn 7 bewegt werden, und von der Geschwindigkeit, mit der die Führungshebel 29, 30 quer zur Endlosbahn 7 bewegt werden.

Die Gummifäden 4a, 4b, 4c der jeweiligen Gruppen, die parallel zur Längsrichtung der Endlosbahn 7 bewegt werden, werden von den Führungshebeln 29, 30 quer zur Endlosbahn 7 zwangsweise bewegt und die Abstände zwischen diesen Gummifäden vergrößern sich, wenn sie von der ersten Position P₁ zur zweiten Position P₂ bewegt werden. Genauer ausgedrückt wird diese Wirkung einerseits durch eine Anordnung erzielt, bei der die bereits erwähnten zylindrischen Führungen 33a, 33b, 33c quer zur Richtung der Bewegung der Endlosbahn 7 parallel im Abstand zueinander angeordnet sind, und andererseits dadurch, daß die Traversiereinrichtungen 21, 22 so gesteuert werden, daß die Kurvenradien der jeweiligen Gummifäden von den in Längsrichtung gegenüberliegenden Enden zu den in Längsrichtung mittleren Bereichen der jeweiligen in Kurven verlaufenden Klebezonen 14 progressiv zunehmen.

Auf diese Weise werden Abschnitte 4A₁, 4B₁ der fadenähnlichen endlosen elastischen Elemente 4A, 4B, die nur auf den jeweiligen Klebezonen verlaufen, von den Klemmwalzen 34, 34 gegen diese gepreßt und dadurch mit der entsprechenden Klebezone 14 verbunden. Abschnitte 4A₂, 4B₂ der fadenähnlichen endlosen elastischen Elemente 4A, 4B, die außerhalb der Klebezonen 14 verlaufen, ziehen sich aufgrund ihrer eigenen Dehnspannung zu einem geradlinigen Zustand zusammen, wobei jedoch eine gewisse Dehnspannung aufrechterhalten bleibt. Dieses Aufrechterhalten der Dehnspannung ist aus nachfolgend erläuterten Gründen von großer Wichtigkeit und kann durch den Dehnungsgrad der fadenähnlichen endlosen elastischen Elemente 4A, 4B und die Kurvenradien der nicht verbundenen Abschnitte 4A₂, 4B₂ gesteuert werden.

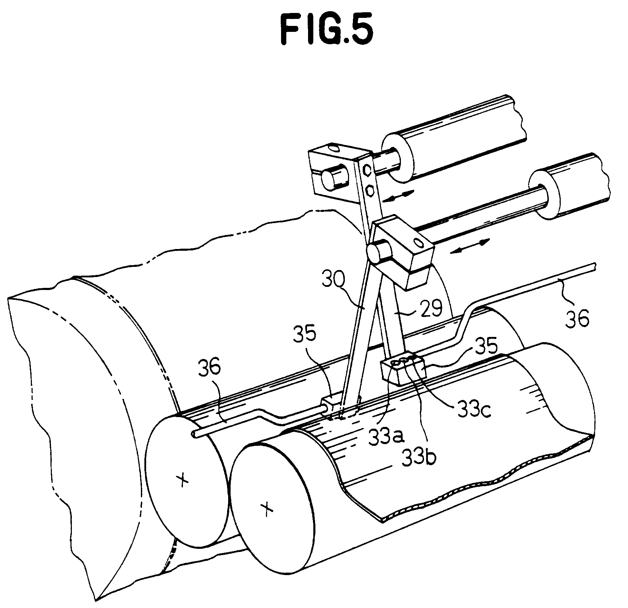

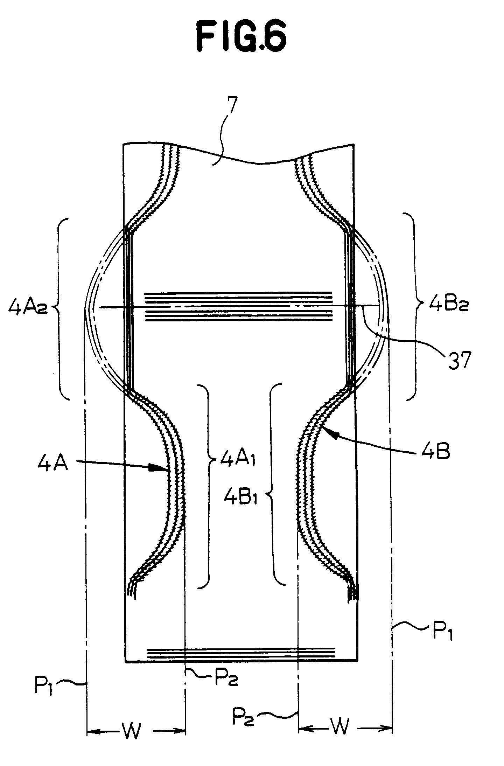

Fig. 5 und 6 zeigen eine perspektivische Darstellung bzw. eine Draufsicht zur Erläuterung teilweiser Veränderungen der Anordnung der in Fig. 3 bzw. 4 gezeigten Traversiereinrichtungen und fadenähnlichen endlosen elastischen Elemente. Bei diesen in Fig. 5 und 6 gezeigten Varianten wird auf die fadenähnlichen endlosen elastischen Elemente 4A, 4B mit Unterbrechungen und direkt Klebstoff aufgebracht.

Wie Fig. 5 zeigt, sind die Führungshebel 29, 30 an ihren unteren Enden mit Klebstoffapplikatoren 35, 35 versehen, die mit den jeweiligen Führungen 33a, 33b, 33c in flüssigkeitsübertragender Verbindung stehen. Diese Applikatoren 35 werden von getrennt vorgesehenen Klebstoffzufuhreinrichtungen (nicht dargestellt) jeweils durch biegsame Schläuche 36, 36 mit Klebstoff unter vorgegebenem Druck beliefert, so daß auf die fadenähnlichen endlosen elastischen Elemente 4A, 4B in deren Längsrichtung mit Unterbrechungen Klebstoff aufgebracht wird. Demgemäß werden, wie in Fig. 6 gezeigt, mit Klebstoff versehene Abschnitte, d. h. die Abschnitte 4A₁, 4B₁ der fadenähnlichen endlosen elastischen Elemente 4A, 4B, die mit der Endlosbahn 7 zu verbinden sind, hergestellt (Abschnitte der jeweiligen Gummifäden 4a, 4b, 4c, die mit kurzen Querstrichen versehen sind, sind jeweils kontinuierlich mit Klebstoff versehen). Wenn gewünscht wird, auf die einzelnen Gummifäden getrennt Klebstoff aufzutragen, kann die von der Anmelderin im US Patent Nr. 4,626,305 aufgezeigte Applikatoreinrichtung (Düsen) verwendet werden.

Das hier durch die vorliegende Erfindung vorgeschlagene Verfahren verwendet die Traversiereinrichtung, um die fadenähnlichen endlosen elastischen Elemente zwangsweise relativ zur Endlosbahn in Kurven zu legen und sie in diesem gekrümmten Zustand mit der Endlosbahn zu verbinden. Dementsprechend müssen die in der vorliegenden Erfindung verwendeten endlosen elastischen Elemente aus fadenähnlichen Einzelelementen aufgebaut sein. Wenn beispielsweise relativ breite Einzelelemente umfassende endlose elastische Elemente bei dem Verfahren gemäß vorliegender Erfindung verwendet werden, neigen die endlosen elastischen Elemente dazu, verdreht zu werden und sich aufgrund unzuverlässiger Verbindung während des Verbindungsvorgangs von der Endlosbahn abzulösen. Ein für das erfindungsgemäße Verfahren geeigneter Querschnitt eines fadenähnlichen endlosen elastischen Elements kann kreisförmig (einschließlich oval), rechteckig oder von anderer unbestimmter Form sein. Es ist jedoch nicht wünschenswert, daß das Verhältnis der größten Querschnittsabmessung zur kleinsten Querschnittsabmessung besonders groß ist.

Die Anbringung des fadenähnlichen elastischen Elements 5 an der Endlosbahn 7 erfolgt unter Verwendung einer bekannten Vorrichtung und eines bekannten Verfahrens zu Herstellung von Wegwerfwindeln. Es ist auch möglich, ein einziges, relativ breites bandähnliches Element anstelle des mehrere Gummifäden umfassenden fadenähnlichen elastischen Elements 5 zu verwenden.

Der vorgeformte Kern 8 wird (in den Figuren nicht gezeigt) zwischen den Abschnitten 4A₁, 4B₁ der in Querrichtung der Endlosbahn 7 einander gegenüberliegenden fadenähnlichen endlosen elastischen Elemente 4A, 4B plaziert, worauf eine weitere Endlosbahn (nicht dargestellt) als Material für die obere Lage 6 dieser Anordnung zugeliefert wird und auf diese oder wenigstens die Endlosbahn 7 auflaminiert wird, wobei eine Klebeschicht dazwischengelegt wird, und schließlich die Teile der Endlosbahnen, die sich außerhalb der Abschnitte 4A₁, 4B₁ erstrecken, abgeschnitten werden, und so ein Endlosschichtkörper gebildet wird.

Der Endlosschichtkörper wird entlang Linien, die die Mittelpunkte der jeweiligen gegenüberliegenden nicht verbundenen Abschnitte 4A₂, 4B₂ der fadenähnlichen endlosen elastischen Elemente 4A, 4B verbinden (entlang den in Fig. 4 und 6 mit Bezugszeichen 37 bezeichneten Linien) quer geschnitten, um so die Rohlinge der einzelnen Kleidungsstücke zu erhalten. Wenn der Endlosschichtkörper in die Rohlinge der einzelnen Kleidungsstücke geschnitten wird, schnappen die nicht verbundenen Abschnitte 4A₂, 4B₂ der fadenähnlichen endlosen elastischen Elemente 4A, 4B durch ihre eigene verbliebene Dehnspannung zurück.

Jedes Kleidungsstück wird entlang einer in Längsrichtung liegenden Mittellinie gefaltet und die in Längsrichtung gegenüberliegenden Enden werden miteinander heißversiegelt, so daß das in Fig. 1 gezeigte Endprodukt entsteht.

Ohne den Umfang der vorliegenden Erfindung zu verlassen ist es auch möglich, die fadenähnlichen endlosen elastischen Elemente an der als obere Lage verwendeten Endlosbahn anstatt an der als untere Lage verwendeten Endlosbahn zu befestigen.

Wie aus vorstehender Beschreibung deutlich wird, erlaubt es die vorliegende Erfindung, fadenähnliche endlose elastische Elemente, die jeweils mehrere fadenähnliche elastische Einzelelemente umfassen, in einfacher Weise um die jeweiligen Beinöffnungen jedes Kleidungsstücks in optimaler Anordnung anzubringen. Genauer bedeutet dies, daß die in gewisser Anzahl vorhandenen fadenähnlichen elastischen Einzelelemente ohne weiteres mit einem Bauteil des einzelnen Kleidungsstücks verbunden werden können, so daß die entsprechenden elastischen Elemente parallel zueinander in Kurven angeordnet sind und die Abstände zwischen ihnen sich von ihren in Längsrichtung entgegengesetzten Enden auf ihre in Längsrichtung mittleren Bereiche (dem Schrittbereich entsprechend) zu progressiv erweitern.

## Patentansprüche

1. Wegwerfbekleidung (1), die um kreisbogenförmige Beinöffnungen (2), die jeweils an gegenüberliegenden Seiten eines zwischen Vorder- und Hinterteilen (10,11) einer oberen und einer unteren Lage (6,7) begrenzten Schrittbereichs (12) gebildet sind, mit elastischen Elementen (4A,4B) in gespanntem Zustand versehen ist, die jeweils mehrere Einzelelemente (4a,4b,4c) umfassen, wobei diese in gewisser Anzahl vorhandenen elastischen Einzelelemente für jede der Beinöffnungen (2) so angeordnet ist, daß die elastischen Einzelelemente in ihrem in Längsrichtung mittleren Bereich den größten Abstand voneinander haben und die Abstände vom mittleren Bereich zu den in Längsrichtung gegenüberliegenden Enden hin nach und nach verringert sind, **dadurch gekennzeichnet,** daß die Dehnspannung der einzelnen, elastischen Elemente (4a,4b,4c) im mittleren Bereich einen maximalen Wert aufweist und an den in Längsrichtung gegenüberliegenden Enden einen minimalen Wert, wobei zwischen den Bereichen ein kontinuierlicher Übergang vorhanden ist.

2. Verfahren zum Anbringen von elastischen Elementen (4A,4B) an Wegwerfbekleidung (1) um jeweilige Beinöffnungen (2), welches die Schritte umfaßt:
kontinuierliches Zuführen von Endlosbahnen, die als Material für eine obere (6) oder untere Lage (7) verwendet werden;
Zuführen von ersten und zweiten fadenähnlichen endlosen elastischen Elementen (4A,4B) in gespanntem Zustand, die jeweils mehrere elastische Einzelelemente (4a,b,c) umfassen, jeweils zu den in Längsrichtung der Endlosbahn verlaufenden gegnüberliegenden Seiten derselben, während das erste und zweite fadenähnliche endlose elastische Element (4A,4B) von Gruppen von parallel im Abstand zueinander angeordneten Führungen (33a,b,c), die jeweils an ersten bzw. an zweiten Traversiereinrichtungen (21,22) vorgesehen sind, gehalten wird;
Hin- und Herbewegen der ersten und zweiten Traversiereinrichtung (21,22) quer zur laufenden Endlosbahn; und
schichtweises Aufbringen von übrigen Teilen als wichtige Bestandteile der Bekleidung auf die Endlosbahn und anschließendes Schneiden der Endlosbahn in Querrichtung gemeinsam mit den ersten und zweiten fadenähnlichen endlosen elastischen Elementen (4A,4B) in jeweilige Abschnitte, **dadurch gekennzeichnet,** daß
Klebstoff mit Unterbrechungen auf die Endlosbahn entlang deren in Längsrichtung verlaufenden gegenüberliegenden Seitenrändern aufgetragen wird, um so bezüglich den jeweiligen Seitenrändern in konkaven Kurven verlaufende, symmetrisch einander gegenüberliegend angeordnete erste und zweite Klebezonen (14) zu bilden und,
daß die erste und zweite Traversiereinrichtung (21,22) quer zur laufenden Endlosbahn derart hin und herbewegt wird, daß die Führungen (33a,b,c) der ersten und zweiten Traversiereinrichtung (21,22) jeweils der ersten bzw. zweiten Klebezone (14) folgen und die Abstände der elastischen Einzelelemente (4a,b,c), die die ersten und zweiten fadenähnlichen endlosen elastischen Elemente (4A,4B) bilden, in den in Längsrichtung mittleren Bereichen bezüglich den in Längsrichtung gegenüberliegenden Enden der ersten und zweiten Klebezone (14) sich erweitern, und das erste und zweite fadenähnliche endlose elastische Element (4A,4B) mit der ersten und zweiten Klebezone (14) verbunden wird, wobei die elastischen Einzelelemente (4a,b,c) parallel zueinander angeordnet sind;
und, daß die Endlosbahn in Querrichtung gemeinsam mit den ersten und zweiten fadenähnlichen endlosen elastischen Elementen (4A,4B) an Stellen in jeweilige Abschnitte geschnitten wird, wo das erste und zweite fadenähnliche endlose elastische Element (4A,4B) nicht mit der Endlosbahn verbunden ist, wodurch Teile der ersten und zweiten fadenähnlichen endlosen elastischen Elemente (4A,4B), die nicht mit der Endlosbahn verbunden sind, sich aufgrund ihrer eigenen Dehnspannung zusammenziehen.

3. Verfahren zum Anbringen von elastischen Elementen (4A,4B) an Wegwerfbekleidung (1) um jeweilige Beinöffnungen (2), welches die Schritte umfaßt:
kontinuierliches Zuführen von Endlosbahnen, die als Material für eine obere (6) oder untere Lage (7) verwendet werden;
Zuführen von ersten und zweiten fadenähnlichen endlosen elastischen Elementen (4A,4B) in gespanntem Zustand, die jeweils mehrere elastische Einzelelemente (4a,b,c) umfassen, jeweils zu den in Längsrichtung der Endlosbahn verlaufenden gegenüberliegenden Seiten derselben; und
Hin- und Herbewegen von ersten und zweiten Traversiereinrichtung (21,22) quer zur bewegten Endlosbahn, so daß die parallel im Abstand zueinander angeordneten Führungen (33a,b,c) der ersten und zweiten Traversiereinrichtung (21,22) jeweils der ersten bzw. zweiten Klebezone (14) folgen, die Abstände der elastischen Einzelelemente (4a,b,c), die die ersten und zweiten fadenähnlichen endlosen elastischen Elemente (4A,4B) bilden, in den in Längsrichtung mittleren Bereichen bezüglich den in Längsrichtung gegenüberliegenden Enden der ersten und zweiten Klebezone (14) sich erweitern, und das erste und zweite fadenähnliche endlose elastische Element (4A,4B) mit der ersten und zweiten Klebezone (14) verbunden wird, wobei die elastischen Einzelelemente parallel zueinander angeordnet sind **gekennzeichnet** durch die folgenden weiteren Verfahrensschritte:
Aufbringen von Klebstoff mit Unterbrechungen auf die ersten und zweiten fadenähnlichen endlosen elastischen Elemente (4A,4B), die von Gruppen von parallel im Abstand zueinander an den ersten bzw. zweiten Traversiereinrichtung (21,22) vorgesehenen Führungen (33a,b,c) gehalten werden, über deren Länge, wobei der Klebstoff von Klebstoffzuführeinrichtungen zugeführt wird, die an der ersten und zweiten Traversiereinrichtung (21,22) nahe an deren jeweiligem vorderen Ende (31,32) gehaltert sind, so daß sie den jeweiligen vorderen Enden folgen; und
schichtweises Aufbringen von übrigen Teilen als wichtige Bestandteile der Bekleidung (1) auf die Endlosbahn und anschließendes Schneiden der Endlosbahn in Querrichtung gemeinsam mit den ersten und zweiten fadenähnlichen endlosen elastischen Elemente (4A,4B) in jeweilige Abschnitte, wo das erste und zweite fadenähnliche endlose elastische Element (4A,4B) nicht mit der Endlosbahn verbunden ist, wodurch Teile der ersten und zweiten fadenähnlichen endlosen elastischen Elemente (4A,4B), die nicht mit der Endlosbahn verbunden sind, sich aufgrund ihrer eigenen Dehnspannung zusammenziehen.

## Claims

1. Disposable garment (1), which is provided about arcuate leg openings (2) with elastic elements (4A, 4B) in the stretched condition, which leg openings are formed respectively on opposite sides of a crotch area (12) defined between forward and rear portions (10, 11) of an upper and of a lower layer (6, 7), said elastic elements (4A, 4B) respectively comprising a plurality of individual elements (4a, 4b, 4c), these elastic individual elements, present in certain numbers, being so disposed for each of the leg openings (2) that the elastic individual elements have the greatest space between them in their central area in the longitudinal direction, and the spacings between the central area and the opposite ends in the longitudinal direction are gradually reduced, characterised in that the expansive tension of the individual elastic elements (4a, 4b, 4c) has a maximum value in the central area, and has a minimum value at the opposite ends in he longitudinal direction, a continuous transition being present between the areas.

2. Method of applying elastic elements 4A, 4B) to disposable garments (1) around respective leg openings (2), comprising the steps:
continuous supply of endless webs which are used as material for an upper (6) or lower layer (7);
supply of first and second thread-like endless elastic elements (4A, 4B) in the tensioned condition, which respectively comprise a plurality of elastic individual elements (4a, 4b, 4c), respectively to the opposite sides of the same extending in the longitudinal direction of the endless web, while the first and second thread-like endless element (4A, 4B) is held by groups of guide means (33a, b, c) disposed in parallel and at a spacing apart from one another, and which respectively are provided on first or on second traversing devices (21, 22);
movement to and fro of the first and second traversing device (21, 22) transversely to the moving endless web; and
application in layers of other parts as important components of the garment on to the endless web and subsequent cutting of the endless web in the transverse direction in common with the first and second thread-like endless elastic elements (4A, 4B) into respective sections, characterised in that
adhesive is applied with interruptions on to the endless web along longitudinally-extending opposite lateral edges, in order in this way to form first and second adhesion zones (14) extending with respect to the respective lateral edges in concave curves and disposed symmetrically opposite one another and
in that the first and second traversing device (21, 22) is moved to and fro transversely to the moving endless web in such a way that the guide means (33a, b, c) of the first and second traversing device (21, 22) respectively follow the first or second adhesion zone (14), and the spaces between the individual elastic elements (4a, b, c) which form the first and second thread-like endless elastic elements (4A, 4B) widen in the central area in the longitudinal direction with respect to the opposite ends, in the longitudinal direction, of the first and second adhesion zone (14), and the first and second thread-like endless elastic element (4A, 4B) is connected to the first and second adhesion zone (14), the individual elastic elements (4a, b, c) being disposed parallel to one another;
and in that the endless web is cut in the transverse direction in common with the first and second thread-like endless elastic elements (4A, 4B) at points into respective sections, where the first and second thread-like element (4A, 4B) is not connected to the endless web, so that portions of the first and second thread-like endless elastic elements (4A, 4B) which are not connected to the endless web, draw together due to their own elastic tension.

3. Method of applying elastic elements (4A, 4B) to disposable garments (1) around respective leg openings (2), comprising the steps:
continuous supply of endless webs used as material for an upper (6) or lower layer (7);
supply of first and second thread-like endless elastic elements (4A, 4B) in the tensioned condition, which respectively comprise a plurality of individual elastic elements (4a, b, c), respectively to the opposite sides of the same extending in the longitudinal direction of three endless web; and
movement to and fro of first and second traversing device (21, 22) transversely to the moving endless web, so that the guide means (33a, b, c) disposed parallel to one another and a distance apart of the first and second traversing device (21, 22) respectively follow the first or second adhesion zone (14), the spaces between the individual elastic elements (4a, b, c) which form the first and second thread-like endless elastic elements (4A, 4B), widen in the central areas in the longitudinal direction with respect to the opposite ends in the longitudinal direction of the first and second adhesion zone (14), and the first and second thread-like endless elastic element (4A, 4B) is connected to the first and second adhesion zone (14), the individual elastic elements being disposed parallel to one another, characterised by the following further process steps:
application of adhesive with interruptions to the first and second thread-like endless elastic elements (4A, 4B), which are held by groups of guide means (33a, b, c) along their length, provided in parallel and at a spacing apart on the first and second traversing device (21, 22), the adhesive being supplied from adhesive supply devices mounted on the first and second traversing device (21, 22) close to their respective forward ends (31, 32), so that they follow the respective forward ends; and
application in layers of other portions as important components of the garment (1) on to the endless web, and subsequent cutting of the endless web in the transverse direction in common with the first and second thread-like endless elastic elements (4A, 4B) into respective sections, where the first and second thread-like endless elastic element (4A, 4B) is not connected to the endless web, so that portions of the first and second thread-like endless elastic elements (4A, 4B) which are not connected to the endless web, draw together due to their inherent tension.

## Revendications

1. Vêtement jetable (1), muni d'éléments élastiques (4A, 4B) à l'état tendu, autour d'ouvertures circulaires (2) de passage des jambes prévues sur des côtés opposés d'une zone d'entre-jambe (12) délimitée entre des parties avant et arrière (10, 11) de couches supérieure et inférieure (6, 7), lesquels éléments élastiques comprennent chacun plusieurs éléments individuels (4a, 4b, 4c), ces éléments élastiques individuels prévus en un certain nombre pour chacune des ouvertures de passage des jambes (2) étant disposés de façon à ce qu'ils présentent le plus grand écartement les uns des autres dans leur zone centrale dans le sens longitudinal, et que les écarts, partant de la zone centrale et allant vers les extrémités opposées dans le sens longitudinal, diminuent petit à petit, caractérisé en ce que la tension d'étirage des divers éléments élastiques (4a, 4b, 4c) présente une valeur maximale dans la zone centrale et une valeur minimale aux extrémités opposées dans le sens longitudinal, une transition continue étant assurée entre les zones.

2. Procédé pour appliquer des éléments élastiques (4A, 4B) sur un vêtement jetable (1), autour d'ouvertures respectives (2) de passage des jambes, comprenant les étapes consistant à :
- amener en continu des bandes continues, utilisées comme matériaux pour une couche supérieure (6) et une couche inférieure (7);
- amener des premiers et des seconds éléments élastiques continus filamentaires (4A, 4B) à l'état tendu, comprenant chacun plusieurs éléments élastiques individuels, en direction des faces longitudinales opposées de la bande continue, tandis que les premier et second éléments élastiques continus filamentaires (4A, 4B) sont maintenus par des guides (33a, b, c) disposés en parallèle et à distance les uns des autres, prévus sur des premier et second dispositifs à mouvement transversal (21, 22);
- animer les premier et second dispositifs à mouvement transversal (21, 22) d'un mouvement de va-et-vient, transversalement à la bande continue en mouvement; et
- appliquer, par couches, les autres parties en tant que composants importants du vêtement, sur la bande continue, et couper ensuite la bande continue dans le sens transversal, en portions individuelles, avec les premier et second éléments élastiques continus filamentaires (4A, 4B), caractérisé en ce que :
- on applique de l'adhésif sur la bande continue, avec des interruptions, le long de ses bords latéraux opposés, dans le sens de la longueur, pour former ainsi des première et seconde zones de collage (14) décrivant des courbes concaves et se faisant symétriquement face par référence aux bords latéraux respectifs, et
- les premier et second dispositifs à mouvement transversal (21, 22) sont animés d'un mouvement de va-et-vient dans le sens transversal de la bande continue tel que les guides (33a, b, c) des premier et second dispositifs à mouvement transversal (21, 22) suivent respectivement la première et la seconde zone de collage (14), et que les écartements des éléments élastiques individuels (4a, b, c), qui forment les premier et second éléments élastiques continus filamentaires (4A, 4B) s'élargissent dans des zones centrales dans le sens de la longueur, par rapport aux extrémités des première et seconde zones de collage (14) opposées dans le sens longitudinal, et que les premier et second éléments élastiques continus filamentaires (4A, 4B) sont raccordés à la première et à la seconde zone de collage (14), les éléments élastiques individuels (4a, b, c) étant disposés parallèlement entre eux,
- et en ce que la bande continue est découpée transversalement en portions respectives, avec les premier et second éléments élastiques continus filamentaires (4A, 4B), à des emplacements où les premier et second éléments élastiques continus filamentaires (4A, 4B) ne sont pas rattachés à la bande continue, ce qui a pour effet de faire se contracter, sous leur tension d'étirage propre, des parties des premier et second éléments élastiques continus filamentaires (4A, 4B) qui ne sont pas rattachées à la bande continue.

3. Procédé pour appliquer des éléments élastiques (4A, 4B) sur un vêtement jetable (1), autour d'ouvertures respectives (2) de passage des jambes, comprenant les étapes consistant à :
- amener en continu des bandes continues, utilisées comme matériaux pour une couche supérieure (6) et une couche inférieure (7);
- amener des premiers et des seconds éléments élastiques continus filamentaires (4A, 4B) à l'état tendu, comprenant chacun plusieurs éléments élastiques individuels, en direction des faces longitudinales opposées de la bande continue; et
- animer les premier et second dispositifs à mouvement transversal (21, 22) d'un mouvement de va-et-vient dans le sens transversal de la bande continue tel que les guides (33a, b, c) montés en parallèle et à distance les uns des autres des premier et second dispositifs à mouvement transversal (21, 22) suivent respectivement la première et la seconde zone de collage (14), que les écartements des éléments élastiques individuels (4a, b, c), qui forment les premier et second éléments élastiques continus filamentaires (4A, 4B) s'élargissent dans des zones centrales dans le sens de la longueur, par rapport aux extrémités des première et seconde zones de collage (14) opposées dans le sens longitudinal, et que les premier et second éléments élastiques continus filamentaires (4A, 4B) sont raccordés à la première et à la seconde zone de collage (14), les éléments élastiques individuels étant disposés parallèlement entre eux, caractérisé par les opérations suivantes :
- application d'adhésif, avec des interruptions, sur les premier et second éléments élastiques continus filamentaires (4A, 4B), qui sont maintenus par des ensembles de guides (33a, b, c) prévus en parallèle et à distance les uns des autres sur les premier et second dispositifs à mouvement transversal (21, 22), sur leur longueur, l'adhésif étant apporté par des dispositifs d'amenée d'adhésif supportés sur les premier et second dispositifs à mouvement transversal (21, 22), à proximité de leurs extrémités avant (31, 32), en sorte qu'ils suivent les extrémités avant respectives; et
- application sur la bande continue, par couches, d'autres parties en tant que composants importants du vêtement (1), suivie d'un découpage de la bande continue transversalement en portions respectives, avec les premier et second éléments élastiques continus filamentaires (4A, 4B), à des emplacements où les premier et second éléments élastiques continus filamentaires (4A, 4B) ne sont pas rattachés à la bande continue, ce qui a pour effet de faire se contracter, sous leur tension d'étirage propre, des parties des premier et second éléments élastiques continus filamentaires (4A, 4B) qui ne sont pas rattachées à la bande continue.
